# DEMANDE DE BREVET EUROPEEN

(11) **EP 2 070 479 A1**
(43) Date de publication de la demande: **17.06.2009**
(21) Numéro de dépôt: 08171459.4
(22) Date de dépôt: 12.12.2008
(51) Int. Cl.: A61B 8/08

(54) **Dispositif d'imagerie échographique et appareil de détection et de destruction de concrétions solides incorporant un tel dispositif**

(30) Priorité: 14.12.2007 FR 0759852
(71) Demandeur: TECHNOMED MEDICAL SYSTEMS, 69120 Vaulx-en-Velin (FR)
(72) Inventeur: Peyrard, André, 42320, Saint-Christo-en-Jarez (FR); Chatre, René, 69200, Venissieux (FR); Nallet, Olivier, 69003, Lyon (FR)
(74) Mandataire: Blanchard, Eugène Gilles

(57) **Abrégé**

La présente invention concerne un dispositif d'imagerie échographique en temps réel, comportant au moins une sonde échographique (**1**) incorporant un transducteur ultrasonique (**2**) connecté électriquement à un générateur de courant pour générer des ondes ultrasonores. Ce dispositif comporte un bras (**3**) d'axe longitudinal X-X' supportant la sonde échographique et étant mobile en translation et en rotation selon son axe longitudinal X-X' par l'intermédiaire de moyens motorisés de déplacement. Il comporte également des moyens de commande des moyens de déplacement et des moyens de contrôle d'effort pour mesurer la pression d'application de la sonde déplacée par le bras mobile contre la surface d'un objet, notamment le corps d'un patient, et des moyens d'asservissement des moyens de déplacement du bras pour maintenir cette pression d'application constante indépendamment des mouvements de la surface de l'objet et des mouvements de la sonde par rapport à cette surface.

## Description

La présente invention concerne un dispositif d'imagerie échographique comportant une sonde d'échographie à pression d'application contrôlée. Elle concerne également un appareil de détection et de destruction de concrétions intracorporelles solides incorporant un tel dispositif d'imagerie échographique, notamment pour des traitements de lithotritie.

On connaît depuis longtemps des dispositifs d'imagerie échographique comportant une sonde formée essentiellement d'un applicateur incorporant un transducteur ultrasonique relié à un générateur de courant pour émettre des ultrasons ainsi que des moyens de réception des ondes ultrasonores réfléchies permettant après filtrage et traitement électronique d'obtenir des images d'un volume dans lequel les ondes ultrasonores ont été transmises et réfléchies. Ces technologies d'imagerie échographiques sont notamment utilisées dans diverses applications médicales pour observer les organes et tissus ou encore les corps étrangers à l'intérieur du corps d'un patient.

Parmi ces applications on compte notamment la lithotritie, qui consiste essentiellement dans la détection et la destruction de lithiases rénales, biliaires ou salivaires par des ondes de choc acoustiques produites par un générateur. Dans cette application, les dispositifs d'imagerie échographiques sont employés, généralement en combinaison avec des moyens d'imagerie par rayons X, pour détecter la présence de lithiases dans le corps d'un patient et leur position, puis suivre au cours du traitement l'évolution de l'état de ces lithiases et leur déplacement éventuel.

Ces mêmes dispositifs peuvent être utilisés pour des applications orthopédiques consistant à délivrer des ondes de choc sur des structures osseuses ou cartilagineuses en vue de traiter des pathologies de type arthrose ou tendinite par exemple. D'autres applications sont en cours d'investigation et la présente invention n'est pas limitée à la seule lithotritie extracorporelle.

Dans tous les cas le principe thérapeutique requiert :
- la mise en correspondance de la cible anatomique, c'est-à-dire de la lithiase avec le foyer actif du générateur d'onde de choc ;
- le suivi, si possible permanent (dit temps réel), de la cible afin de pouvoir détecter un défaut de correspondance résultant du mouvement de la cible en cours de traitement (mouvement inopiné du patient, mouvement respiratoire naturel etc. ....).

Les deux technologies d'imagerie précitées sont couramment utilisées conjointement car elles sont complémentaires pour la fonction de mise en correspondance de la cible avec le foyer du générateur du fait que certains calculs peuvent être soit radio- soit écho- transparents et doivent donc être localisé par la technologie qui permet de les visualiser.

De plus, pour la fonction de suivi, qui nécessite idéalement une visualisation permanente du calcul, la technologie échographique s'impose par son innocuité, notamment par l'absence de rayonnements ionisants. Dans tous les cas le principe thérapeutique requiert l'optimisation de la qualité des images.

La qualité des images échographiques obtenues dépend en grande partie du couplage acoustique entre la sonde et le patient. Aux fréquences utilisées dans le domaine médical (MHz), les ondes ultrasonores ne se transmettent pas dans l'air, et les examens échographiques ont pendant longtemps, et sont encore dans une majorité de cas, réalisés par un médecin qui supporte manuellement la sonde échographique et l'applique sur le corps d'un patient.

Il est très fréquent que, pour améliorer le couplage ou optimiser les angles de vues et la qualité des images, les médecins utilisent un gel à base d'eau qui permet d'assurer une bonne transmission des ondes acoustiques ultrasonores au contact du patient. Il est également très fréquent qu'ils fassent également varier la pression de contact de la sonde sur la peau du patient. Dans certains cas, par exemple d'exploration profonde ou d'exploration d'organes difficile d'accès, cette pression peut être suffisamment élevée pour provoquer une gêne chez le patient.

La pression d'application de la sonde échographique doit également être adaptée en fonction du patient examiné. Par exemple, un enfant ou un patient maigre n'aura pas la même tolérance à l'effort de contact de la sonde échographique qu'un adulte ou un patient de forte corpulence.

Enfin, l'application manuelle de l'examen échographique est handicapante et fatigante pour les médecins, notamment pour des examens et traitements longs comme peuvent l'être les traitements de lithotritie.

Le brevet WO 90/01904 décrit un système mécanique qui permet le suivi des mouvements d'une cible, notamment un calcul rénal, par des moyens d'imagerie échographique dans un appareil de lithotritie.

Dans le système décrit dans le document WO 90/01904, le support de la sonde échographique est réalisé par un dispositif mécanique sans contrôle par l'opérateur. Ce dispositif mécanique permet de positionner et d'appliquer la sonde échographique sur le corps d'un patient pour réaliser une échographie de repérage d'une lithiase. Le générateur d'ondes de choc de l'appareil de lithotritie décrit dans ce document ayant une position fixe horizontale dans l'espace, les efforts exercés par les forces de gravité sur la sonde échographique et par conséquent les efforts exercés sur un patient sont donc constants.

Toutefois, si le patient vient à bouger légèrement ou que l'on déplace la sonde, la qualité des images échographiques obtenues est particulièrement affectée. De même selon que le patient est maigre ou obèse, la pression de de la sonde au contact du patient sera plus ou moins importante et la qualité des images échographiques également affectée.

Le dispositif mécanique de support de la sonde acoustique décrit dans WO 90/01904 ne permet malheureusement pas dans de tels cas de rétablir rapidement et correctement un couplage et donc des images satisfaisantes et s'avèrent donc pour certains cas moins efficace ou moins pratique qu'une manipulation manuelle de la sonde échographique, notamment pour suivre le déplacement d'une lithiase dans le corps.

De plus, dans les appareils de lithotritie de générations récentes, la pression d'application de la sonde échographique (l'effort de contact) peut varier au cours d'un même examen, du fait de la mobilité en rotation du générateur d'ondes de choc qui est monté sur un support mobile. Alors, selon la position angulaire du générateur les forces de gravité sur la sonde échographique varient et donc la pression d'application de la sonde sur le patient évolue également dans le sens d'une pression positive ou négative non constante avec un impact négatif sur la qualité des images échographiques obtenues.

Il existe donc un problème technique important à résoudre consistant à automatiser de façon efficace l'action de déplacement et d'application d'une sonde échographique contre le corps du patient pour obtenir le meilleur couplage et la meilleure image échographique possible quelles que soient la morphologie du patient et la position de la sonde par rapport à celui-ci.

Le problème technique susvisé inclut notamment la difficulté d'assurer de façon automatisée un maintien d'une pression positive constante de la sonde échographique contre le corps du patient pour assurer le couplage de la sonde tout en permettant au praticien de choisir et de faire varier en fonction des patients et de la position de la sonde échographique la valeur de la pression d'application de la sonde sur le patient.

Ces problèmes sont notamment importants dans le domaine des traitements de lithotritie du fait de la nécessité de s'assurer de conserver en permanence en correspondance la position des lithiases traitées avec le foyer des générateurs d'ondes de choc quelle que soit la position de ces générateurs.

Le but de la présente invention est en conséquence de procurer une solution au problème technique susvisé.

Le but de l'invention est également de procurer une solution au problème technique ci-dessus évoqué qui soit simple et efficace à mettre en oeuvre pour les opérateurs/médecins.

Le but de l'invention est également de procurer une solution au problème technique précédemment évoqué qui puisse être facilement appliquée dans les différentes applications médicales et vétérinaires d'imagerie échographique.

Ces différents buts sont atteints conformément à la présente invention par la fourniture d'un dispositif d'imagerie échographique, comportant au moins une sonde échographique incorporant un transducteur ultrasonique connecté électriquement à un générateur de courant pour générer des ondes ultrasonores. Ce dispositif se caractérise par le fait qu'il comporte un bras droit d'axe longitudinal X-X' supportant la sonde échographique et qui est mobile en translation et en rotation selon son axe longitudinal X-X' par l'intermédiaire de moyens motorisés de déplacement.

Ce dispositif se caractérise également par le fait qu'il comporte des moyens électriques de commande des moyens motorisés de déplacement du bras mobile ainsi que des moyens de contrôle d'effort pour mesurer la pression d'application de la sonde déplacée par le bras contre la surface d'un objet, notamment le corps d'un patient.

Le dispositif d'imagerie échographique selon l'invention comporte enfin des moyens d'asservissement des moyens de déplacement du bras mobile pour maintenir cette pression d'application constante indépendamment des mouvements de la surface de l'objet sur lequel la sonde échographique est appliquée et des mouvements de la sonde par rapport à cette surface.

Le dispositif d'imagerie échographique selon l'invention permet ainsi d'assurer de façon simple et performante l'application d'une sonde échographique sur la surface d'un objet, notamment le corps d'un patient, de façon automatisée et à pression constante. En effet, grâce au moyen de contrôle d'effort le dispositif calcul en permanence la pression d'application de la sonde échographique. Les moyens de contrôle d'effort sont avantageusement reliés aux moyens de commandes des moyens de déplacement du bras mobile du dispositif portant la sonde ou même directement à ces moyens de déplacement pour ajuster, par l'intermédiaire des moyens d'asservissement, leur fonctionnement de façon à conserver une pression d'application de la sonde constante.

Il convient de noter ici qu'il est possible de configurer le dispositif d'imagerie de l'invention de telle façon que la régulation de la pression d'application de la sonde échographie par le bras mobile soit dépendante ou non de la position de la sonde dans l'espace, et donc des forces de gravité.

En effet, on peut choisir de réaliser l'asservissement des moyens de déplacement motorisés du bras mobile par rapport à une valeur de pression d'application de consigne, auquel cas seule la valeur de pression donnée par les moyens de contrôle d'effort pilote l'asservissement qui se fait indépendamment de la position de la sonde échographique.

On peut également de façon avantageuse choisir de réaliser l'asservissement des moyens de déplacement motorisés du bras mobile par rapport à une valeur de courant ou de tension de consigne pour l'alimentation desdits moyens de déplacement motorisés. Cette valeur de courant/tension est choisie comme correspondant à une pression d'application de la sonde souhaitée, auquel cas alors la position spatiale de la sonde échographique et les forces de gravité interviennent et sont prises en compte automatiquement dans la régulation de la pression d'application de la sonde échographique par son bras mobile.

Un autre objet de la présente invention consiste également à procurer un appareil de détection et de destruction de concrétions intracorporelles solides telles que des lithiases rénales, biliaires ou salivaires qui comporte un dispositif d'imagerie échographique selon l'invention tel que défini ci-dessus. Un tel appareil peut également trouver une application dans le domaine de l'orthopédie dans lequel les ondes de choc sont alors utilisées à plus faible puissance pour le traitement des douleurs articulaires.

Cet appareil comporte alors dans sa plus simple réalisation un bâti fixe supportant des moyens d'imagerie de concrétions intracorporelles localisées dans le corps d'un individu ou d'un animal, ces moyens d'imagerie comportant au moins le dispositif d'imagerie échographique de l'invention, et un générateur d'ondes de choc acoustiques configuré pour atteindre et détruire lesdites concrétions intracorporelles localisées dans le corps d'un individu ou d'un animal à partir de la localisation desdites concrétions intracorporelles par les moyens d'imagerie.

Diverses autres caractéristiques ressortent de la description faite ci-dessous en référence aux dessins annexés qui montrent, à titre d'exemples non limitatifs, des formes de réalisation de l'objet de l'invention et parmi lesquels :
- la **figure 1** représente, en perspective, le dispositif d'imagerie échographique de l'invention dans un mode préféré de réalisation, associé à un générateur d'ondes de choc d'un appareil de détection et destruction de lithiases ;
- la **figure 2** est une vue de droite du dispositif d'imagerie de l'invention associé à un générateur d'ondes de choc d'un appareil de détection et destruction de lithiases, montrant les différents degrés de mobilité en rotation du dispositif par rapport à un centre de rotation immatériel **O** confondu avec le second foyer **F2** du générateur d'ondes ;
- la **figure 3** est une vue analogue à celle de la **figure 2**, montrant une position inclinée du dispositif d'imagerie de l'invention suite à un déplacement de l'ensemble formé par le dispositif d'imagerie et le générateur d'ondes de choc ;
- la **figure 4** est une vue de dessus et en perspective du dispositif d'imagerie échographique de l'invention, associé à un générateur d'ondes de choc d'un appareil de détection et destruction de lithiases ;
- la **figure 5** représente schématiquement la structure du bras mobile de support de la sonde échographique du dispositif d'imagerie de l'invention et ses facultés de réglage de position et de longueur de course selon l'axe longitudinal X-X' du bras ;
- la **figure 6** est une vue analogue à la **figure 5** montrant une seconde position de réglage de la longueur de course du bras mobile de la sonde échographique du dispositif d'imagerie de l'invention.

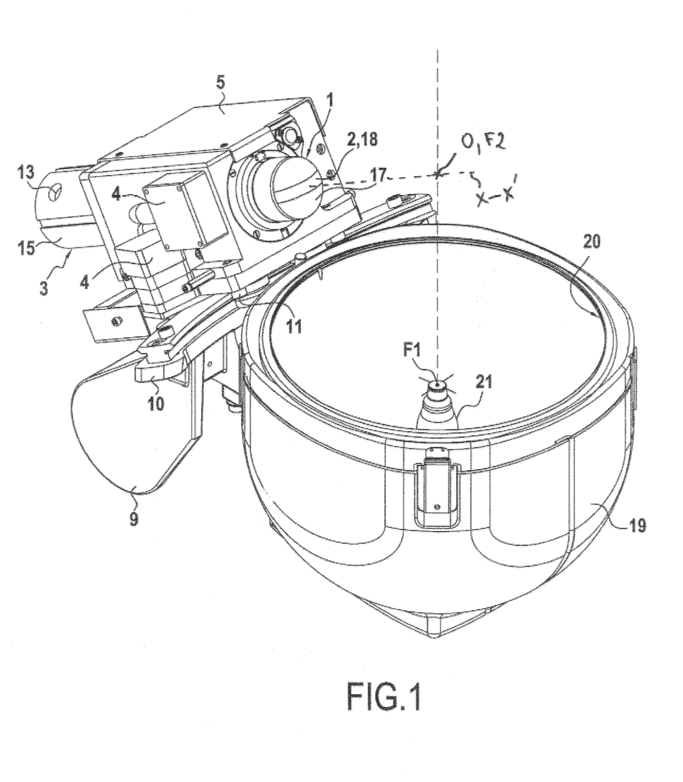

Les **figures 1** à **4** représentent sous diverses vues le dispositif d'imagerie échographique selon la présente invention associé à un générateur d'ondes de choc tel que communément utilisé dans les appareils de détection et de destruction de lithiases à l'intérieur du corps d'un être humain ou d'un animal. Bien que le dispositif d'imagerie de l'invention ne soit pas réservé ni destiné spécifiquement à une telle application de visualisation et de destruction de lithiases, il trouve un intérêt tout particulier d'utilisation dans cette application et c'est en référence à celle-ci que la description qui va suivre est réalisée.

Le dispositif d'imagerie échographique de l'invention comporte avantageusement, dans le mode de réalisation préféré représenté, une sonde échographique **1** incorporant un transducteur ultrasonique 2 connecté électriquement, de façon traditionnelle, à un générateur de courant (non représenté) pour générer des ondes ultrasonores permettant lorsque la sonde **1** est appliquée contre le corps d'un patient d'obtenir une image échographique du plan du corps dans lequel se répandent les ondes ultrasonores.

Conformément à l'invention, la sonde échographique **1** est portée par un bras **3** droit, d'axe longitudinal X-X', mobile en translation et en rotation selon cet axe X-X' par l'intermédiaire de moyens motorisés **4** de déplacement. Le bras mobile **3** est monté coulissant sur un châssis **5** sur lequel sont également fixés les moyens motorisés **4** de déplacement du bras **3**.

Le dispositif d'imagerie de l'invention comporte également des moyens électriques de commande des moyens motorisés **4** de déplacement ainsi que des moyens de contrôle d'effort pour mesurer la pression d'application de la sonde déplacée par le bras mobile contre la surface du corps d'un patient.

Les moyens de contrôle d'effort peuvent notamment être constitués de capteurs d'efforts disposés sur le bras **3**, ou dans la sonde échographique **1** elle-même ou encore associés aux moyens motorisés de déplacement du bras **3**. De tels capteurs d'efforts peuvent par exemple être constitués de jauges de contraintes ou encore de capteur de type FSR™ (acronyme de Force Sensing Resistors en anglais).

En complément des moyens de commande et des moyens de contrôle d'efforts, le dispositif de l'invention comporte également des moyens d'asservissement des moyens de déplacement motorisés **4** du bras **3** de support de la sonde échographique **1**. Ces moyens d'asservissement permettent, en coopération avec les moyens de commande, les moyens de contrôle d'effort et les moyens motorisés **4** de déplacement du bras mobile, de maintenir la pression d'application de la sonde échographique **1** constante indépendamment des mouvements du corps du patient et des mouvements de la sonde sur ce corps.

Conformément à une caractéristique préférée du dispositif de l'invention, les moyens de déplacements motorisés du bras mobile **3** comportent des moteurs **4** du type servo-moteurs tels que les moteurs référencés Coolmuscle, de la société Myostat Motion (Canada). Ces moteurs **4** comportent de façon avantageuse des moyens de contrôle et d'asservissement de leur tension et/ou de leur courant d'alimentation par rapport à une valeur de consigne déterminée en fonction de la pression d'application de la sonde échographique à procurer. En particulier, ces moteurs incorporent des microprocesseurs programmables qui peuvent remplir simultanément les fonctions de contrôle d'effort et d'asservissement et tension et/ou courant des moteurs afin d'ajuster et de réguler les mouvements du bras **3** et la pression d'application de la sonde échographique **1** sur le corps d'un patient.

Ainsi grâce à de tels moteurs programmables **4**, les moyens de contrôle d'effort et les moyens d'asservissement peuvent intégrés aux moyens motorisés **4** de déplacement du bras mobile **3** portant la sonde **1**.

En variante, les moyens de contrôle d'effort et d'asservissement peuvent également être intégrés aux moyens de commande, lesquels de façon classique dans le domaine de l'appareillage médicale, comportent des moyens informatiques et logiciels.

Ces moyens informatiques et logiciels permettent entre autre un réglage et un contrôle de la pression d'application de la sonde échographique **4** à procurer sur le corps d'un patient, en particulier en fonction de la morphologie dudit patient. Ils sont également aptes à piloter les moyens de déplacements motorisés **4** du bras mobile **3** supportant la sonde **1** de sorte que celle-ci soit appliquée sur le corps du patient à la pression adéquate pour obtenir un bon couplage entre la sonde et le corps du patient et ainsi de bonnes images échographiques, lesquelles sont également obtenues et traités par des moyens informatiques et logiciels bien connus dans le domaine des techniques échographiques.

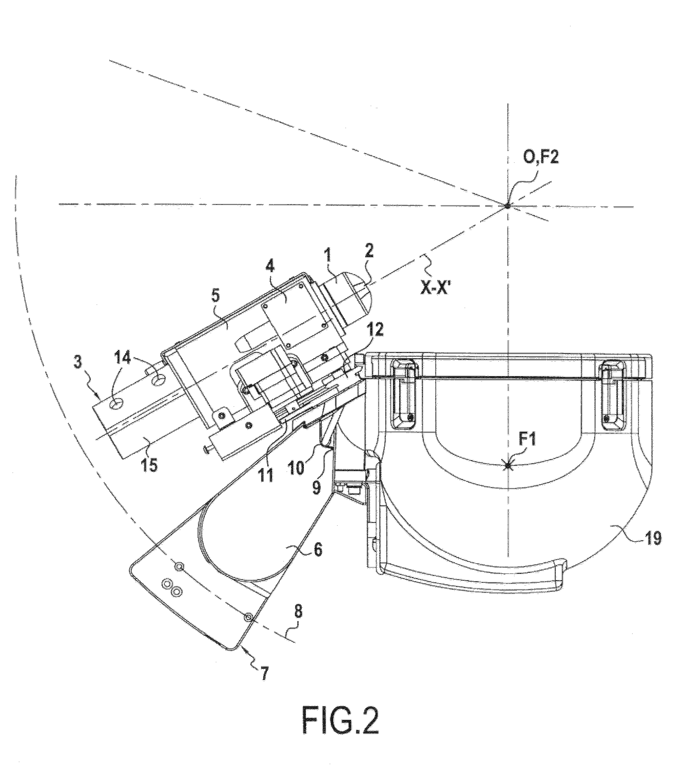

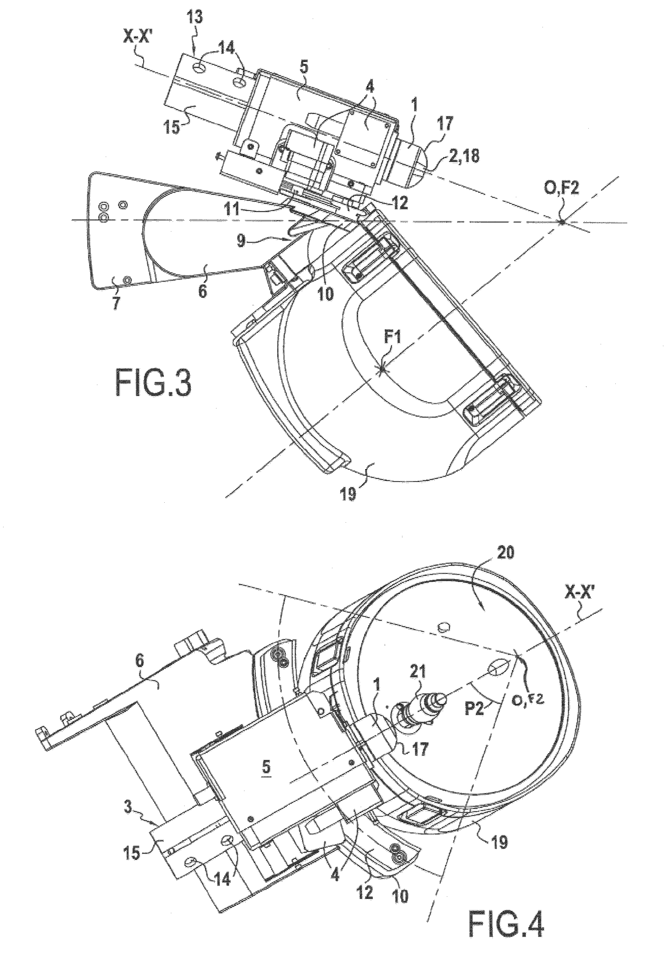

Comme cela ressort en particulier sur les figures **2** et **3**, le châssis **5** sur lequel est monté le bras mobile **3** portant la sonde **1** est lui-même mobile en rotation au moins dans un premier plan **P1** contenant l'axe X-X' du bras **3** et dans un second plan **P2** sensiblement perpendiculaire au plan **P1,** par rapport à un même centre de rotation immatériel **O** situé sur l'axe longitudinal X-X' du bras **3** mobile portant la sonde **1**.

Cette mobilité dans les plans **P1** et **P2** perpendiculaires autour du même centre de rotation immatériel **O** est en particulier permise, dans l'exemple représenté, par le fait que le dispositif d'imagerie de l'invention comporte un étrier **6** de support monté coulissant par une première extrémité **7** munie de moyens de fixation appropriés sur un rail de guidage circulaire **8**, matérialisé sur la figure **2** par un arc de cercle en traits d'axes, de façon à mener le bras mobile **3** et la sonde échographique **1** en rotation dans le plan **P1** par rapport au centre de rotation immatériel **O.**

De préférence, le débattement en rotation dans le plan **P1** de l'étrier couvre un secteur angulaire d'au moins 50° par rapport à **O.**

Par ailleurs, comme cela ressort de la figure **4**, l'étrier **6** supporte également solidairement à sa seconde extrémité **9** une platine **10** supportant un chariot **11** sur lequel est fixé le châssis 5 sur lequel sont fixés le bras mobile et la sonde échographique. Le chariot **11**, comme visible sur les figures **1** et **3** notamment, est lui monté coulissant sur un rail de guidage circulaire **12** formé ou fixé sur la platine **10** de façon à mener le bras droit mobile **3** et la sonde **1** en rotation dans le plan **P2** perpendiculaire à **P1** par rapport au centre de rotation immatériel **O.**

Dans le plan **P2**, il est souhaitable que le chariot permette une couverture d'un secteur angulaire de 30° au moins par rapport à **O.**

Afin de se déplacer le long des rails de guidage **8** et **12**, l'étrier **6** et le chariot **11** comportent avantageusement des moyens de déplacement motorisés, non représentés sur les figures, reliés aux moyens électriques de commande du dispositif de l'invention et aptes à coopérer avec les dits rails de guidage circulaires **8, 12**.

Ces moyens de déplacement motorisés de l'étrier **6** et du chariot **11** portant le châssis **5** permettent avantageusement de manipuler et déplacer automatiquement la sonde échographique **1** et le bras **3** qui la porte et la déplace en rotation autour du point **O** de façon automatique par rapport au corps d'un patient, sans que ce dernier n'ait à bouger sur le matelas ou la table sur lequel il est allongé pendant l'examen ni que le praticien réalisant l'examen n'ait lui-même à déplacer la sonde **1** pour modifier l'axe de visée de celle-ci.

Ainsi le dispositif d'imagerie échographique de l'invention permet-il un déplacement automatisé de la sonde échographique selon trois rotations distinctes et selon une translation selon l'axe X-X' ce qui autorise un positionnement rapide et précis de la sonde, avec la pression d'application nécessaire et suffisante pour obtenir un couplage et des images satisfaisantes avec une bonne résolution sans gène pour le patient ni opération manuelle du praticien de l'examen sur le patient. La valeur de chacun de ces déplacements est mesurée afin de connaître la position de la sonde dans l'espace par rapport à un référentiel quelconque dont l'origine peut être le point **O.**

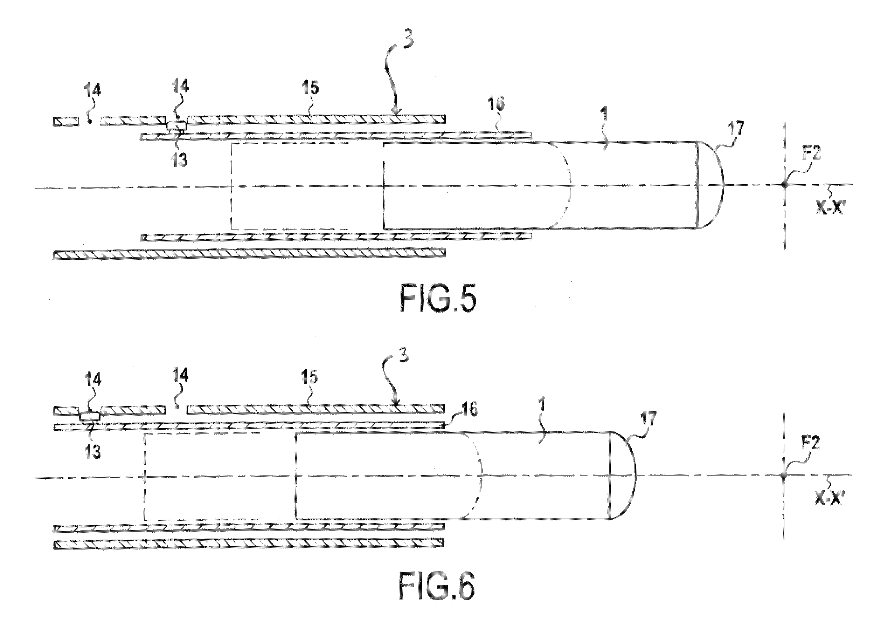

Conformément à une autre caractéristique avantageuse et comme représenté sur les figures **5** et **6**, le dispositif d'imagerie de la présente invention comporte des moyens de réglage **13, 14** de la position du bras mobile **3** portant la sonde échographique **1** par rapport au châssis **5** et de la longueur de sa course en translation selon son axe longitudinal X-X'.

Ces moyens de réglage ont en particulier pour vocation de permettre une adaptation de la longueur du bras **3** et de sa course le long de l'axe X-X' afin de pouvoir réaliser un examen échographique de tout type de patient, enfant ou adulte, maigre ou obèse.

Pour ce faire, dans le mode de réalisation préféré représenté aux figures **5** et **6**, le bras mobile **3** comporte au moins deux sections de tube **15, 16** télescopiques et coulissantes l'une par rapport à l'autre, la section de tube **15** formant un fourreau à l'intérieur duquel est réglable la position de la seconde section de tube **16** qui porte elle la sonde échographique **1**. Afin d'opérer un blocage en translation des deux sections de tubes **15, 16** l'une par rapport à l'autre et donc de permettre un réglage de la position du bras **3** et de la longueur de sa course en translation, les moyens de réglage comportent au moins un moyen de blocage mâle **13** et au moins un moyen de blocage femelle **14** formés respectivement sur chacune des sections **15**, 16 de tubes télescopiques du bras mobile. Par exemple, comme représenté sur les figures **5, 6**, le moyen de blocage mâle **13** peut être constitué d'un plot ou bouton poussoir fixé sur la section de tube **16** apte à coopérer avec au moins un, et de préférence au moins deux moyens de blocage femelle **14** formés par des orifices percés dans le fourreau **15** du bras **3**.

Ainsi, lorsque le plot ou bouton **13** sur la section **16** est enfoncé, il est possible de faire coulisser la section **16** du bras **3** dans le fourreau 15 pour allonger ou réduire la longueur du bras **3** et modifier la position « de base » de la sonde échographique **1** et par conséquent ajuster la longueur de course en translation du bras **3** selon l'axe X-X'. Lorsque le plot **13** sur la section **16** se trouve en correspondance avec un des orifices **14** sur le fourreau **15**, celui-ci remonte alors dans l'orifice **14** sous l'effet d'un ressort, réalisant ainsi le blocage des deux sections télescopiques **15, 16** du bras **3** l'une par rapport à l'autre.

Ce mécanisme de bras à double sections **15, 16** permet d'obtenir une offset mécanique choisi par le praticien selon la corpulence du patient.

Les moyens motorisés **4** précédemment décrits sont ceux qui permettent le déplacement de la sonde **1** dans le fourreau **16** pour permettre le contrôle de la pression d'application de la sonde au contact du patient.

Par ces simples dispositions, le dispositif d'imagerie de l'invention permet avantageusement aux praticiens d'ajuster la longueur du bras **3** portant la sonde échographique à la morphologie de chaque patient pour assurer au mieux la qualité du couplage entre la sonde et le corps du patient.

Enfin, dans la forme de réalisation préférée ici représentée du dispositif d'imagerie échographique de l'invention, celui-ci comporte également sur la sonde échographique une cupule d'application **17** sphérique ou hémisphérique pour procurer à la sonde une surface d'application dépourvue de discontinuités ou d'arêtes. Cette cupule **17** permet de faciliter le contact et le glissement de la sonde sur le patient lors des ajustements en position de la sonde lors des déplacements du bras portant celle-ci en translation et/ou en rotation selon son axe X-X' ainsi que en rotation dans les plans **P1** et **P2**.

La cupule **17** peut notamment se clipser sur l'extrémité de la sonde. Elle peut être jetable, et donc destinée à un usage unique, ou bien lavable et réutilisable.

Elle présente avantageusement au moins une zone **18** transparente aux ultrasons, de préférence disposée en regard du transducteur ultrasonore **2** et de forme complémentaire de celui-ci. Cette zone transparente **18** peut notamment être formée par une ouverture de forme complémentaire du transducteur échographique de la sonde **1.**

Cette cupule **17**, outre sa fonction d'aide au glissement et au couplage de la sonde sur le corps des patients, constitue également enfin un élément de sécurité rendu nécessaire par l'asservissement automatique de la pression d'application de la sonde **1** permis par le dispositif de l'invention et qui échappe au contrôle des praticiens.

Le dispositif d'imagerie échographique de l'invention tel que décrit ci-avant est essentiellement dédié à des applications médicales. Il a notamment été conçu tout particulièrement pour répondre aux besoins des praticiens dans les traitements de lithotritie extracorporelle des lithiases rénales, biliaires ou salivaires ou structures osseuses ou cartilagineuses.

Ces traitements nécessitent en particulier l'utilisation de moyens d'imagerie échographique pour repérer et suivre en temps réel les lithiases et leur évolution dans le corps des patients au cours du traitement.

Dans ce cadre, la présente invention propose également un appareil de détection et de destruction de concrétions intracorporelles solides qui incorpore un dispositif d'imagerie échographique tel que précédemment décrit et représenté aux figures **1** à **6**.

Le dispositif d'imagerie échographique de l'invention permet une visualisation permanente du calcul et donc notamment un suivi en temps réel des concrétions traitées en assurant en permanence, grâce au bras mobile **3** portant la sonde échographique **1** dont la pression d'application sur le corps du patient est contrôlée et asservie automatiquement, un couplage idéal de la sonde échographique **1** sur le corps du patient, indépendamment des mouvements parasites éventuelles du patient ou de la sonde.

L'appareil de détection et de destruction de concrétions intracorporelles de l'invention comporte de façon traditionnelle un bâti fixe supportant des moyens d'imagerie de concrétions intracorporelles localisées dans le corps d'un individu ou d'un animal ainsi qu'un générateur d'ondes de choc acoustiques (ou ondes de pression) représenté sur les figures **1** à **4** et désigné par la référence **19** sur ces figures. De façon classique, ce générateur **19** est configuré pour émettre des ondes de choc acoustiques dirigées vers un foyer cible **F2** que l'on cherche à mettre en coïncidence, par l'intermédiaire des moyens d'imagerie, avec les concrétions pour atteindre et détruire celles-ci.

Les moyens d'imagerie de l'appareil de détection et de destruction de concrétions intracorporelles de l'invention comportent comme évoqué ci-dessus un dispositif d'imagerie échographique conforme à la présente invention. Additionnellement et de façon préférée et traditionnelle, ces moyens d'imagerie comportent également de préférence des moyens d'imagerie par rayons X.

L'association de moyens d'imagerie par rayons X et du dispositif d'imagerie échographique de l'invention trouve sa pertinence notamment dans le fait que certaines lithiases sont radio-transparentes ou bien écho-transparentes et il est donc nécessaire pour les repérer d'avoir à sa disposition les moyens d'imagerie le permettant. Les moyens d'imagerie rayons X délivrent aussi des images parfois plus faciles à interpréter que celle obtenues par échographie. Souvent l'information obtenue par une modalité d'imagerie trouve avantage à être confirmée par l'autre modalité.

De préférence et comme représenté sur les figures **1** à **4**, le générateur d'ondes de choc acoustiques **19** comporte un réflecteur ellipsoïdal **20** dans lequel est positionnée une électrode **21** qui permet de générer une onde de choc acoustique au premier foyer **F1** du réflecteur ellipsoïdal **20**. Par la géométrie particulière du réflecteur **20**, l'onde de choc produite en **F1** par l'électrode **21** se trouve réfléchie au second foyer **F2** du réflecteur **20**, lequel foyer **F2** se trouve à l'extérieur du générateur, ce qui permet de viser des concrétions situées dans le corps d'un patient ou d'un animal en mettant en correspondance ces concrétions et le foyer **F2** du générateur.

Conformément à une caractéristique de l'appareil de détection et de destruction de concrétions intracorporelles solides de l'invention, le dispositif d'imagerie échographique est monté solidaire du bâti de l'appareil de sorte que le centre de rotation immatériel **O,** qui se trouve sur l'axe longitudinal X-X' de la sonde échographique **1** et de son bras mobile **3**, coïncide avec le second foyer **F2** du réflecteur du générateur d'ondes de choc.

En particulier, comme représenté sur les figures **1** à **4**, il est particulièrement avantageux que le générateur **19** d'ondes de choc soit fixé au niveau de la seconde extrémité **9** de l'étrier **6** du dispositif d'imagerie échographique de façon à être mobile en rotation simultanément à la sonde échographique **1** et au bras mobile **3** la portant dans le plan **P1** par rapport au second foyer **F2** du générateur d'ondes de choc confondu avec le centre de rotation immatériel **O.**

Ainsi l'appareil de l'invention permet-il de déplacer simultanément en rotation autour du centre de rotation immatériel **O,** confondu avec le foyer cible **F2** lui-même en coïncidence avec une concrétion à détruire dans le corps d'un patient, la sonde échographique **1** et le générateur **19** afin de suivre en temps réel les concrétions dans le corps du patient. Grâce à la mobilité automatique du bras **3** de la sonde échographique **1** et la maîtrise également automatique de la pression d'application de cette sonde sur le corps permis par le dispositif d'imagerie de l'invention, il est ainsi possible d'obtenir en continu les meilleures images échographiques des concrétions traitées et de leur position dans le corps du patient tout en ayant toujours le générateur **19** dans une position de tir adéquate.

La rotation du générateur **19** autour du point immatériel **O** permet de rechercher la meilleure voie d'abord des ondes de choc produites par celui-ci sur les concrétions à détruire en permettant d'éviter des structures tissulaires à préserver ou des structures osseuses faisant obstacle aux ultrasons. L'appareil selon l'invention procure de façon avantageuse la possibilité pour le praticien réalisant l'examen de faire apparaître sur les images échographiques obtenues grâce au dispositif d'imagerie échographique le foyer cible **F2** ainsi que le cône de focalisation des ondes de choc. La liaison en rotation de la sonde échographique **1** et du générateur **19** permet de conserver la même incidence relative du cône de focalisation par rapport à l'axe d'imagerie échographique X-X'.

Pour que la mise en coïncidence du foyer cible **F2** du générateur avec la ou les concrétions à détruire dans le corps d'un individu ou d'un animal soit aisée et surtout que le patient traité soit dans une position la plus confortable possible pendant son traitement, l'appareil de l'invention comporte de façon classique une table (non représentée) de repos et de positionnement du corps par rapport au générateur d'onde de choc **19**.

De façon avantageuse, ladite table est équipée, conformément à la présente invention, de moyens de déplacement adaptés pour déplacer le corps du patient traité sans que celui-ci n'ait à bouger de manière à positionner une concrétion intracorporelle à traiter exactement en correspondance avec le second foyer **F2** du générateur d'ondes de choc **19**.

Ces moyens de déplacement de la table de l'appareil peuvent notamment comprendre des vérins électriques, pilotés par des moyens de commande électriques manuels ou bien informatisés pour déplacer la table dans trois directions orthogonales X, Y, Z de l'espace.

De tels vérins permettent notamment, après détection de la position de la concrétion à viser par l'intermédiaire des moyens d'imagerie de l'appareil, que se soient la sonde échographique ou les moyens d'imagerie radiographique par rayons X, de déplacer automatiquement la table supportant le corps du patient traité pour positionner celui-ci par rapport au générateur de sorte qu'une concrétion à détruire soit exactement positionnée au foyer cible **F2** du générateur **19**.

Bien entendu, au cours de ce déplacement de la table, le praticien réalisant le traitement peut suivre en direct sur des écrans de contrôle projetant les images obtenues par les moyens d'imagerie la mise en correspondance du foyer **F2** avec la concrétion à détruire et le cas échéant ajuster manuellement par l'intermédiaire d'une télécommande manuelle ou bien encore par des moyens de commande tactile sur les écrans, le positionnement de la table ainsi que du générateur **19** pour que la fenêtre de tir soit la meilleure possible.

L'appareil de détection et de destruction de concrétions intracorporelles solides de l'invention qui comprend un dispositif d'imagerie échographique conforme à l'invention permet donc une optimisation de la qualité des images échographiques obtenues dans les traitements de lithotritie extracorporelle ainsi que du confort et de la simplicité de mise en oeuvre pour le praticien et de traitement pour le patient.

L'invention n'est pas limitée aux exemples décrits et représentés car diverses modifications peuvent y être apportées sans sortir de son cadre.

## Revendications

1. Dispositif d'imagerie échographique, comportant au moins une sonde échographique (**1**) incorporant un transducteur ultrasonique (**2**) connecté électriquement à un générateur de courant pour générer des ondes ultrasonores, **caractérisé en ce qu'**il comporte un bras (**3**) droit d'axe longitudinal X-X' supportant la sonde échographique (**1**) et étant mobile en translation et en rotation selon son axe longitudinal X-X' par l'intermédiaire de moyens motorisés (**4**) de déplacement, et étant monté sur un châssis (5) mobile en rotation au moins dans un premier plan **P1** et dans un second plan **P2** sensiblement perpendiculaire au plan **P1** par rapport à un même centre de rotation immatériel **O** situé sur l'axe longitudinal X-X' du bras mobile (**3**) portant la sonde (**1**), le centre de rotation immatériel **O** et l'axe longitudinal X-X' étant contenus dans les plans **P1** et **P2**, et **en ce qu'**il comporte des moyens électroniques de commande des moyens motorisés (**4**) de déplacement ainsi que des moyens de contrôle d'effort pour mesurer la pression d'application de la sonde (**1**) déplacée par le bras mobile (**3**) contre la surface d'un objet, notamment le corps d'un patient, et des moyens d'asservissement des moyens de déplacement du bras (**3**) pour maintenir cette pression d'application constante indépendamment des mouvements de la surface de l'objet et des mouvements de la sonde (**1**) par rapport à cette surface.

2. Dispositif selon la revendication 1, **caractérisé en ce qu'**il comporte un étrier (**6**) de support monté coulissant par une première extrémité (**7**) munie de moyens de fixation appropriés sur un rail de guidage circulaire (**8**) de façon à mener la sonde échographique (**1**) et le bras mobile (**3**) la portant en rotation dans le plan **P1** par rapport au centre de rotation immatériel **O,** ledit étrier supportant solidairement à sa seconde extrémité (**9**) une platine (**10**) supportant un chariot (**11**) sur lequel est fixé le châssis (**5**) sur lequel sont fixés le bras mobile (**3**) et la sonde échographique (**1**), le chariot (**11**) étant monté coulissant sur un rail de guidage circulaire (**12**) formé sur la platine (**10**) de façon à mener le bras droit mobile (**3**) et la sonde (**1**) en rotation dans le plan **P2** par rapport au centre de rotation immatériel **O.**

3. Dispositif selon la revendication 2, **caractérisé en ce que** l'étrier (**6**) et le chariot (**11**) comportent des moyens de déplacement motorisés reliés aux moyens électroniques de commande et aptes à coopérer avec les rails de guidage circulaire (**8, 12**) de l'étrier et du chariot respectivement.

4. Dispositif selon l'une des revendications 1 à 3, **caractérisé en ce que** les moyens de contrôle d'effort et les moyens d'asservissement sont intégrés aux moyens motorisés (**4**) de déplacement du bras mobile (**3**) portant la sonde (1) et/ou aux moyens de commande.

5. Dispositif selon l'une des revendications 1 à 4, **caractérisé en ce qu'**il comporte des moyens de réglage (**13, 14**) de la position du bras mobile (**3**) portant la sonde échographique (**1**) et de la longueur de sa course en translation selon son axe longitudinal X-X'.

6. Dispositif selon la revendication 5, **caractérisé en ce que** le bras mobile (**3**) comporte au moins deux sections de tube (**15, 16**) télescopiques coulissante l'une par rapport à l'autre et **en ce que** les moyens de réglage de la position du bras et de la longueur de course en translation du bras mobile comportent au moins un moyen de blocage mâle (**13**) et au moins un moyen de blocage femelle (**14**) formés respectivement sur chacune des sections de tubes télescopiques du bras mobile.

7. Dispositif selon l'une des revendications 1 à 6, **caractérisé en ce que** les moyens de déplacements du bras mobile (**3**) en translation et rotation selon son axe longitudinal X-X' comportent des moteurs programmables (**4**) comportant des moyens de contrôle et d'asservissement de leur tension et/ou de leur courant d'alimentation par rapport à une consigne déterminée en fonction de la pression d'application de la sonde échographique (**1**) à procurer.

8. Dispositif selon l'une des revendications 1 à 7, **caractérisé en ce que** les moyens de commandes comportent des moyens informatiques et logiciels de réglage et de contrôle de la pression d'application de la sonde échographique (**1**) à procurer sur la surface d'un objet et aptes à piloter les moyens de déplacements motorisés (**4**) du bras mobile (**3**) supportant la sonde (**1**).

9. Dispositif selon l'une des revendications 3 à 8, **caractérisé en ce que** les moyens de commandes sont aptes à piloter les moyens de déplacement de l'étrier (**6**) et du chariot (**11**) pour activer les mouvements de rotation du châssis (**5**) dans les plans P**1** et P**2** en rotation autour du centre de rotation immatériel O.

10. Dispositif selon l'une des revendications 1 à 9, **caractérisé en ce que** la sonde échographique comporte une cupule d'application (**17**) sphérique ou hémisphérique présentant au moins une zone (**18**) transparente aux ultrasons disposée en regard du transducteur ultrasonore (**2**) et de forme complémentaire de celui-ci pour procurer à la sonde une surface d'application dépourvue de discontinuités ou d'arêtes.

11. Appareil de détection et de destruction de concrétions intracorporelles solides telles que des lithiases rénales, biliaires, salivaires, ou structures osseuses ou cartilagineuses comportant un bâti fixe supportant des moyens d'imagerie de concrétions intracorporelles localisées dans le corps d'un individu ou d'un animal et un générateur (**19**) d'ondes de choc acoustiques configurés pour atteindre et détruire desdites concrétions intracorporelles localisées dans le corps d'un individu ou d'un animal, **caractérisé en ce que** les moyens d'imagerie comporte un dispositif d'imagerie échographique selon l'une des revendications 1 à 10.

12. Appareil de détection et de destruction de concrétions intracorporelles solides selon la revendication 11, **caractérisé en ce que** le générateur (**19**) d'ondes de choc acoustiques comporte un réflecteur ellipsoïdal (**20**) et une électrode (**21**) positionnée dans le réflecteur de manière à générer une onde de choc acoustique au premier foyer **F1** du réflecteur pour que cette onde soit réfléchie au second foyer **F2** du réflecteur et **en ce qu'**il comporte un dispositif d'imagerie échographique selon l'une des revendications 2 à 11 solidaire du bâti de l'appareil de sorte que le centre de rotation immatériel **O** coïncide avec le seconde foyer **F2** du réflecteur du générateur d'ondes de choc.

13. Appareil de détection et de destruction de concrétions intracorporelles solides selon l'une des revendications 11 ou 12, **caractérisé en ce qu'**il comporte une table de repos et de positionnement du corps d'un individu ou d'un animal par rapport au générateur d'onde de choc, ladite table étant équipée de moyens de déplacement adaptés pour déplacer le corps d'un individu ou d'un animal de manière à positionner une dite concrétion intracorporelle exactement au second foyer **F2** du générateur d'ondes de choc.

14. Appareil de détection et de destruction de concrétions intracorporelles solides selon l'une des revendications 11 à 13, **caractérisé en ce que** les moyens d'imagerie comporte également des moyens d'imagerie par rayons X.

15. Appareil de détection et de destruction de concrétions intracorporelles solides selon l'une des revendications 11 à 14, **caractérisé en ce qu'**il comporte un dispositif d'imagerie échographique selon l'une des revendications 2 à 10 et **en ce que** le générateur (**19**) d'ondes de choc est fixé au niveau de la seconde extrémité (**9**) de l'étrier (**6**) du dispositif d'imagerie échographique de façon à être mobile en rotation simultanément à la sonde échographique (1) et au bras mobile (**3**) la portant dans le plan **P1** par rapport au second foyer F2 du générateur d'ondes de choc confondu avec le centre de rotation immatériel **O.**
